(19) Europäisches Patentamt
European Patent Office
Office européen des brevets

(11) Veröffentlichungsnummer : **0 023 647**
**B1**

(12) # EUROPÄISCHE PATENTSCHRIFT

(45) Veröffentlichungstag der Patentschrift : **22.09.82**

(51) Int. Cl.³ : **C 07 C 63/70**, C 07 C 51/58

(21) Anmeldenummer : **80104232.6**

(22) Anmeldetag : **18.07.80**

(54) **Verfahren zur Herstellung von halogenierten Benzoylfluoriden.**

(30) Priorität : **04.08.79 DE 2931688**

(43) Veröffentlichungstag der Anmeldung : **11.02.81 (Patentblatt 81/06)**

(45) Bekanntmachung des Hinweises auf die Patenterteilung : **22.09.82 Patentblatt 82/38**

(84) Benannte Vertragsstaaten : **BE CH DE FR GB IT LI NL**

(56) Entgegenhaltungen :
**US A 2 181 554**
**US A 3 560 553**

(73) Patentinhaber : **BAYER AG**
**Zentralbereich Patente, Marken und Lizenzen**
**D-5090 Leverkusen 1, Bayerwerk (DE)**

(72) Erfinder : **Marhold, Albrecht, Dr.**
**Carl-Duisberg-Strasse 329**
**D-5090 Leverkusen 1 (DE)**
Erfinder : **Klauke, Erich, Dr.**
**Eichendorffweg 8**
**D-5068 Odenthal (DE)**

**EP 0 023 647 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von halogenierten Benzoylfluoriden

Die vorliegende Erfindung betrifft ein Verfahren zur Herstellung von halogenierten Benzoylfluoriden.

Es ist bekannt, daß aus Pentafluorbenzotrifluorid bei einwöchigem Erhitzen mit Schwefelsäure unter Rückfluß Pentafluorbenzoesäure in 25 %iger Ausbeute erhalten wird (J. Am. Chem. Soc. 73, 1366, (1951)).

Es wurde nun ein Verfahren zur Herstellung von halogenierten Benzoylfluoriden gefunden, das dadurch gekennzeichnet ist, daß man ein halogeniertes Benzotrifluorid der Formel

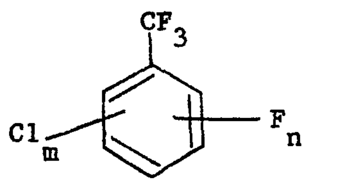

(I),

in der

m und n unabhängig voneinander für eine ganze Zahl von 0 bis 5 stehen, wobei die Summe von m und n 5 ist, mit mindestens einer Schwefel-Verbindung der Formel

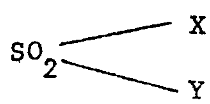

(II),

in der

X Fluor, Chlor oder Hydroxyl bedeutet,

Y für Hydroxyl steht oder

X und Y gemeinsam ein durch eine Doppelbindung mit dem Schwefelatom verbundenes Sauerstoffatom bedeuten, bei erhöhter Temperatur umsetzt, die Schwefel-Verbindung nach der Umsetzung mit dem halogenierten Benzotrifluorid in Form einer Verbindung der Formel

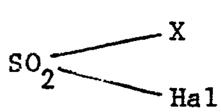

(III),

in der

X die genannte Bedeutung hat und

Hal für Fluor oder Chlor steht, aus dem Reaktionsgemisch entfernt und gegebenenfalls das Reaktionsprodukt mit Kaliumfluorid bei erhöhter Temperatur und erhöhtem Druck weiter umsetzt.

Als halogenierte Benzotrifluoride für das erfindungsgemäße Verfahren seien fünffach im Kern durch Fluor, Chlor oder durch Fluor und Chlor substituierte Benzotrifluoride genannt, wie Pentachlorbenzotrifluorid, Pentafluorbenzotrifluorid, Tetrachlor-2-fluorbenzotrifluorid, Tetrachlor-3-fluor-benzotrifluorid, Tetrachlor-4-fluorbenzotrifluorid, die isomeren Trichlor-difluorbenzotrifluoride, die isomeren Dichlor-trifluorbenzotrifluoride und die isomeren Chlor-tetrafluor-benzotrifluoride.

Als Beispiele für Schwefel-Verbindungen der Formel (II) für das erfindungsgemäße Verfahren seien genannt: Fluorsulfonsäure, Chlorsulfonsäure, Schwefelsäure oder Schwefeltrioxid. Die genannten Schwefel-Verbindungen können einzeln oder im Gemisch eingesetzt werden. Bevorzugt ist der Einsatz von Chlorsulfonsäure oder Oleum, dem Gemisch aus Schwefelsäure und Schwefeltrioxid. Für den Fall, daß Oleum eingesetzt wird, sei ein solches mit einer Konzentration von beispielsweise 0,1 bis 65 Gew.-% $SO_3$, bevorzugt ein solches mit 10 bis 40 Gew.-% $SO_3$, genannt.

Die Menge an Schwefel-Verbindung der Formel (II) beträgt zur Erzielung einer möglichst hohen Ausbeute mindestens 1 Mol pro Mol eingesetztes halogeniertes Benzotrifluorid. Bevorzugt wird eine Menge von 1 bis 3 Mol Schwefel-Verbindung pro Mol halogeniertes Benzotrifluorid eingesetzt. Eine größere Menge an Schwefel-Verbindung als die angegebene ist für den Erfolg des erfindungsgemäßen Verfahrens unkritisch, jedoch aus wirtschaftlichen Gründen wenig vorteilhaft.

Als Temperatur für das erfindungsgemäße Verfahren sei eine solche von 70 bis 150 °C, bevorzugt 80 bis 130 °C, genannt.

Im erfindungsgemäßen Verfahren wird die eingesetzte Schwefel-Verbindung der Formel (II) nach der Umsetzung mit dem halogenierten Benzotrifluorid in Form einer Verbindung der Formel (III) aus dem Reaktionsgemisch entfernt. Die Entfernung aus dem Reaktionsgemisch kann beispielsweise durch Destillation erfolgen. Als Verbindungen der Formel (III) seien beispielsweise genannt: Sulfurylfluorid, Sulfurylchlorid, Sulfurylchloridfluorid, Fluorsulfonsäure oder Chlorsulfonsäure. Im Falle der Verwendung von Schwefelsäure, Schwefeltrioxid oder Oleum, dem Gemisch aus Schwefelsäure und Schwefeltrioxid, als Schwefel-Verbindung der Formel (II) kann es vorkommen, daß diese Schwefel-Verbindung nach Beendigung der erfindungsgemäßen Umsetzung mit einem halogenierten Benzotrifluorid nicht oder nicht vollständig als Verbindung der Formel (II) vorliegt.

In diesem Fall ist es erforderlich, zur Entfernung der Schwefel-Verbindung in Form der Verbindung der Formel (III), dem Reaktionsansatz nach Beendigung der erfindungsgemäßen Umsetzung mit dem halogenierten Benzotrifluorid ein Säurehalogenid des Kohlenstoffs, Phosphors oder Schwefel zuzusetzen. Als solche Säurehalogenide seien beispielsweise die Säurefluoride oder die Säurechloride, bevorzugt die Säurechloride, genannt. Beispiele für geeignete Säurechloride sind Phosgen, Phosphortrichlorid oder Thionylchlorid, bevorzugt Thionylchlorid.

Für den Fall, daß ein höherer Grad der Fluorierung im aromatischen Kern gewünscht wird, als

dem Fluorierungsgrad des eingesetzten halogenierten Benzotrifluorids entspricht, oder für den Fall, daß durch den erfindungsgemäßen Einsatz eines Säurechlorids des Kohlenstoffs, Phosphors oder Schwefels, beispielsweise Thionylchlorid, zur Umwandlung der Schwefel-Verbindung der Formel (II) in eine Verbindung der Formel (III) die gebildete Benzoylfluoridgruppe ganz oder teilweise in eine Benzoylchloridgruppe übergeführt wurde, kann das erfindungsgemäße Reaktionsprodukt mit Kaliumfluorid bei erhöhter Temperatur und erhöhtem Druck weiter umgesetzt werden.

Für jeden Chlor-Fluor-Austausch ist zur Erzielung einer möglichst hohen Ausbeute mindestens 1 Mol Kaliumfluorid erforderlich. Die Gesamtmenge an einzusetzendem Kaliumfluorid richtet sich daher vornehmlich nach der Anzahlauszutauschender Chloratome pro Mol halogenierten Benzoylfluorids. So ist es beispielsweise günstig, zur Umwandlung eines Pentachlorbenzoylfluorids in ein Pentafluorbenzoylfluorid 6 bis 7 Mol Kaliumfluorid pro Mol des Pentachlorbenzoylfluorids einzusetzen Eine höhere als die genannte Menge Kaliumfluorid ist nicht nachteilig, ergibt jedoch im allgemeinen keine erhöhte Ausbeute und ist daher aus wirtschaftlichen Gründen unzweckmäßig. Sollen beispielsweise von 5 am aromatischen Kern gebundenen Chloratomen nur 4 gegen Fluor ausgetauscht werden, ist es zweckmäßig, 4 Mol Kaliumfluorid pro Mol halogeniertes Benzoylfluorid einzusetzen. Bei dieser Verfahrensweise tritt wenig Pentafluorbenzoylfluorid auf, jedoch als Hauptprodukt das Tetrafluorchlorbenzoylfluorid neben nur wenig minderfluoriertem Produkt. Das erhaltene Fluorierungsgemisch kann gut durch Destillation getrennt werden, wobei das gewünschte Tetrafluor-chlorbenzoylfluorid als fertiges Produkt anfällt, während die minderfluorierten Produkte in das Ausgangsmaterial des erfindungsgemäßen Verfahrens zurückgeführt werden können. Wenn man in einer anderen Verfahrensvariante etwa 3 Mol Kaliumfluorid pro Mol Pentachlorbenzoylfluorid einsetzt, wird die Bildung des Pentafluorbenzoylfluorids fast völlig unterdrückt. Das Tetrafluor-chlor-benzoylfluorid tritt in untergeordneter Menge auf, als Hauptmenge jedoch das Trifluor-dichlor-benzoylfluorid. Auch bei dieser Verfahrensvariante ist eine gute destillative Trennung möglich.

Das Kaliumfluorid kann zur Steigerung seiner Fluorierungsaktivität mit einem höheren Alkalifluorid, beispielsweise Rubidiumfluorid oder Cäsiumfluorid, bevorzugt Cäsiumfluorid, versetzt werden. Als Cäsiumfluorid-Menge im Kaliumfluorid sei beispielsweise 0,1 bis 99,9 Mol-%, bevorzugt 0,2 bis 10 Mol-% besonders bevorzugt 0,5 bis 5 Mol-%, des Gemisches an Kaliumfluorid und Cäsiumfluorid genannt.

Der Chlor-Fluor-Austausch kann ohne oder mit Lösungsmittel durchgeführt werden. Die Ausführungsform ohne Lösungsmittel kann etwas höhere Reaktionstemperaturen erforderlich machen und eventuell die Ausbeute verschlechtern. Daher ist die Ausführungsform mit Lösungsmittel bevorzugt. Als Lösungsmittel sei beispielsweise Dimethylsulfon, N-Methyl-pyrrolidon oder Tetramethylsulfon, bevorzugt Tetramethylsulfon, genannt.

Der Chlor-Fluor-Austausch in der bevorzugten Form in Gegenwart eines Lösungsmittels wird beispiels-weise bei einer Temperatur von 170 bis 280 °C, bevorzugt 180 bis 270 °C, durchgeführt. Eine höhere Temperatur innerhalb des genannten Temperaturbereiches kommt hierbei in Betracht, wenn ein hoher Fluorierungsgrad erreicht werden soll. Umgekehrt kann die Temperatur innerhalb des angegebenen Bereiches etwas niedriger sein, wenn ein niedrigerer Fluorierungsgrad erzielt werden soll.

Der Chlor Fluor-Austausch wird bei erhöhtem Druck, beispielsweise bei 3 bis 20 bar, bevorzugt 5 bis 15 bar, ausgeführt. Dieser Druck kann beispielsweise durch den Eigendruck des Reaktionsgemisches erreicht werden. Es kann jedoch auch günstig sein, den gewählten Druck durch ein Inertgas, beispielsweise Stickstoff oder Argon, einzustellen.

Die halogenierten Benzoylfluoride werden im erfindungsgemäßen Verfahren überraschenderweise durch einfache Umsetzungen mit allgemein verfügbaren Chemikalien und in guten Ausbeuten erhalten.

Die im erfindungsgemäßen Verfahren erhältlichen halogenierten Benzoylfluoride sind wertvolle Zwischenprodukte für die Herstellung von Pflanzenschutzmitteln. So können sie beispielsweise durch Erwärmen auf 50 °C mit etwa äquimolaren Mengen Natriumborhydrid in Dioxan als Lösungsmittel zu den halogenierten Benzylalkoholen reduziert werden (DE-OS 26 58 074). Nach der gleichen DE-OS lassen sich diese halogenierten Benzylalkohole beispielsweise durch Reaktion mit einem substituierten Cyclopropancarbonsäurechlorid bei 70 bis 120 °C zu dem entsprechenden Benzylester der entsprechenden substituierten Cyclopropancarbonsäure umsetzen, die ihrerseits wertvolle Wirkstoffe zur Bekämpfung von tierischen Pflanzenschädlingen, insbesondere Insekten und Spinnentieren, darstellen.

Beispiel 1

a) In einer Apparatur aus Edelstahl werden 128 g Pentachlorbenzotrifluorid und 145 g Chlorsulfonsäure vorgelegt. Unter Rühren wird auf 80 °C erwärmt, wobei die Entwicklung von Fluorwasserstoff einsetzt. Beim Nachlassen der Gasentwicklung steigert man noch die Temperatur auf 115 °C, kühlt anschließend ab und destilliert bei 15 mbar die überschüssige Chlorsulfonsäure, sowie entstandene Sulfurylhalogenide, ab. Anschließend erhält man durch Destillation im Hochvakuum 88 g Pentachlorbenzoylfluorid (76 % der theoretischen Ausbeute) ; Fp : 118 °C.

b) In einem Autoklaven aus Edelstahl legt man 1 184 g Pentachlorbenzoylfluorid, 1 625 g trocke-

nes Kaliumfluorid und 2 800 ml Tetramethylsulfon vor und stellt durch Aufpressen von trockenem Stickstoff einen Druck von 8 bar ein. Dann erhitzt man unter Rühren und unter dem sich einstellenden Eigendruck 4 Stunden lang bei 250 °C, kühlt ab und entspannt. Der Reaktionsansatz wird von den anorganischen Salzen abfiltriert und das Filtrat über eine Kolonne destilliert. Man erhält 86 g Pentafluorbenzoylfluorid (Kp : 40 bis 44 °C/16 mbar) und 568 g einer Mischung (Kp : 60 bis 90 °C/16 mbar), die aus Tetrafluorchlorbenzoylfluorid (63 Gew.-%) und Trifluordichlorbenzoylfluorid (37 Gew.-%) besteht, die entweder destillativ in die Komponenten getrennt werden können oder zur erneuten Fluorierung eingesetzt werden können

Beispiel 2

Eine Mischung aus 520 g 3,5-Dichlor-2,4,6-trifluorbenzotrifluorid und 400 g Chlorsulfonsäure wird, wie im Beispiel 1 beschrieben, für 4 Stunden unter Rühren erhitzt. Durch Destillation erhält man 392 g 3,5-Dichlor-2,4,6-trifluorbenzoylfluorid (82 % der theoretischen Ausbeute) ; Kp : 90 bis 95 °C/15 mbar ; $n_D^{20}$ : 1 500 7.

Beispiel 3

a) In einer Apparatur aus Edelstahl, die mit Rührer, Rückflußkühler, Dosiereinrichtung und Gasableitung in einen Wäscher ausgestattet ist, werden 2 700 g Pentachlorbenzotrifluorid vorgelegt und auf 130 °C erwärmt. Dann tropft man 1 080 g 35 Gew.-%iges Oleum innerhalb von 30 Minuten zu und rührt ca. 4 Stunden bis zum Ende der Gasentwicklung nach. Danach kühlt man auf ca. 75 °C und tropft 1 474 g Thionylchlorid zu. Nach weiteren 4 Stunden und beendeter Gasentwicklung ersetzt man den Rückflußkühler durch eine Destillationseinrichtung und destilliert bis zu einer Innentemperatur von 120 °C überschüssige Reagenzien und Nebenprodukte ab. Anschließend wird bei einem Vakuum von etwa 20 mbar bei 150 °C Innentemperatur eine Mischung aus chlorsulfonsäure und Fluorsulfonsäure abdestilliert. Bei weiterer Destillation bei einem Druck von 0,1 mbar werden 2 437 g eines Gemisches aus Pentachlorbenzoylfluorid und Pentachlorbenzoylchlorid (94 % der theoretischen Ausbeute) erhalten, Kp : 140 bis 150 °C/0,1 mbar.

b) In einem Autoklaven aus Edelstahl werden 2 400 g der unter a) erhaltenen Mischung mit 3 200 g trockenem Kaliumfluorid und 5 700 ml Tetramethylensulfon für 4 Stunden unter einem Stickstoffdruck von 10 bar bei 250 °C gerührt, anschließend abgekühlt und entspannt. Der Reaktionsansatz wird durch Filtration von den anorganischen Salzen befreit und bei einem Druck von 20 mbar bis zu einer Übergangstemperatur von 148 °C von wenigen Verunreinigungen abdestilliert. Das aufgefangene Destillat, das auch das gesamte Tetramethylensulfon enthält, wird mit 680 g trockenem Kaliumfluorid erneut bei 250 bis 260 °C und 10 bar Stickstoffdruck für 4 Stunden gerührt. Durch erneute Destillation werden 487 g Pentafluorbenzoylfluorid und 208 g Tetrafluorchlorbenzoylfluorid erhalten.

Beispiele für die Weiterverarbeitung

Beispiel 4

Man legt 38 g Natriumborhydrid in 200 ml trockenem Dioxan bei 20 °C vor und tropft unter Rühren eine Lösung aus 86 g Pentafluorbenzoylfluorid in 100 ml Dioxan zu. Nach dem Ende der Zugabe rührt man für 2 Stunden bei 20 °C und für 30 Minuten bei 80 °C nach, kühlt ab und tropft langsam 75 ml und anschließend verdünnte Salzsäure bis zur deutlich sauren Reaktion zu. Nach dem Extrahieren mit Methylenchlorid wird die organische Phase destilliert. Man erhält bei einem Kp : 87 °C/19 mbar eine Menge von 168 g Pentafluorbenzylalkohol.

Beispiel 5

In einem 2-1-Dreihalskolben mit Rührer, Rückflußkühler und Tropftrichter werden 400 ml trockenes Dioxan und 55 g Natriumborhydrid bei 20 °C vorgelegt und sodann 247 g 3,5-Dichlor-2,4,6-trifluorbenzoylfluorid, gelöst in 250 ml trockenem Dioxan, bei dieser Temperatur zugetropft. Nach dem Ende der Zugabe wird noch für 2 Stunden bei 20 °C gerührt, anschließend für 1 Stunde auf Rückflußtemperatur erwärmt. Die auf Raumtemperatur abgekühlte Lösung wird sodann tropfenweise mit insgesamt 50 ml Wasser versetzt und anschließend mit verdünnter Salzsäure sauer gestellt. Man extrahiert die Reaktionsmischung dreimal mit Methylenchlorid und destilliert das Lösungsmittel nach Trocknung über Natriumsulfat ab. Aus dem Rückstand erhält man durch weitere Destillation 196,5 g (85 % der theoretischen Ausbeute) 3,5-Dichlor-2,4,6-trifluorbenzylalkohol vom Kp : 134 bis 135 °C/20 mbar ; Fp : 67 bis 68 °C.

**Ansprüche**

1. Verfahren zur Herstellung von halogenierten Benzoylfluoriden, dadurch gekennzeichnet, daß man ein halogeniertes Benzotrifluorid der Formel

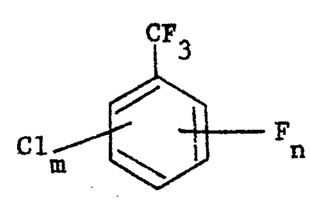

in der

m und n unabhängig voneinander für eine ganze Zahl von 0 bis 5 stehen, wobei die Summe von m und n 5 ist, mit mindestens einer Schwefel-Verbindung der Formel

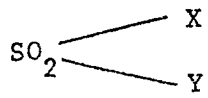

in der

X Fluor, Chlor oder Hydroxyl bedeutet,

Y für Hydroxyl steht oder

X und Y gemeinsam ein durch eine Doppelbindung mit dem Schwefelatom verbundenes Sauerstoffatom bedeuten, bei erhöhter Temperatur umsetzt, die Schwefel-Verbindung nach der Umsetzung mit den halogenierten Benzotrifluorid in Form einer Verbindung der Formel

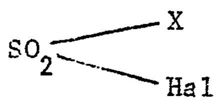

in der

X die genannte Bedeutung hat und

Hal für Fluor oder Chlor steht, aus dem Reaktionsgemisch entfernt und gegebenenfalls das Reaktionsprodukt mit Kaliumfluorid bei erhöhter Temperatur und erhöhtem Druck weiter umsetzt.

2. Verfahren nach Anspruch 1, dadurch gekennzeichnet, daß man als Schwefel-Verbindung Chlorsulfonsäure einsetzt.

3. Verfahren nach Anspruch 1, dadurch gekennzeichnet, daß man als Schwefel-Verbindung ein Gemisch aus Schwefelsäure und Schwefeltrioxid einsetzt, dieses Gemisch der Schwefel-Verbindungen nach der Umsetzung mit dem halogenierten Benzotrifluorid mit einem Säurehalogenid des Kohlenstoffs, Phosphors oder Schwefels in eine Verbindung der Formel

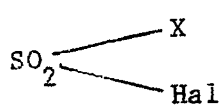

in der

X und Hal die in Anspruch 1 angegebene Bedeutung haben, umwandelt und als solche aus dem Reaktionsgemisch entfernt.

4. Verfahren nach Anspruch 1, dadurch gekennzeichnet, daß man Pentachlorbenzotrifluorid mit Chlorsulfonsäure bei 100 bis 130 °C umsetzt und das entstandene Pentachlorbenzoylfluorid anschließend mit Kaliumfluorid in Tetramethylensulfon bei erhöhter Temperatur und erhöhtem Druck weiter umsetzt.

5. Verfahren nach Anspruch 1 und 3, dadurch gekennzeichnet, daß man Pentachlorbenzotrifluorid mit einem Gemisch aus Schwefelsäure und Schwefeltrioxid umsetzt und die Schwefelsäure und das Oleum, sowie ihre Umsetzungsprodukte nach Reaktion mit Thionylchlorid in Form von Verbindungen der Formel

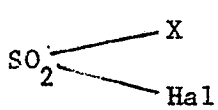

in der

X und Hal die in Anspruch 1 angegebene Bedeutung haben, aus dem Reaktionsgemisch durch Destillation entfernt.

**Claims**

1. Process for the preparation of halogenated benzoyl fluorides, characterised in that a halogenated benzotrifluoride of the formula

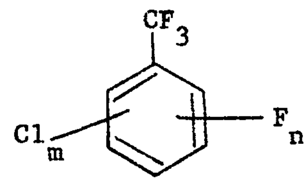

in which

m and n independently of one another represent an integer from 0 to 5, the sum of m and n being 5, is reacted with at least one sulphur compound of the formula

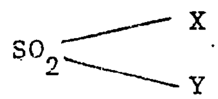

in which

X denotes fluorine, chlorine or hydroxyl and

Y represents hydroxyl, or

X and Y together denote an oxygen atom linked to the sulphur atom by a double bond, at elevated temperature, and after reaction with the halogenated benzotrifluoride, the sulphur compound is removed from the reaction mixture in the form of a compound of the formula

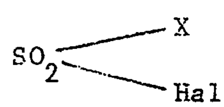

in which

X has the meaning given and

Hal represents fluorine or chlorine, and, if appropriate, the reaction product is further reacted with potassium fluoride at elevated temperature and under increased pressure.

2. Process according to Claim 1, characterised in that chlorosulphonic acid is used as the sulphur compound.

3. Process according to Claim 1, characterised in that a mixture of sulphuric acid and sulphur trioxide is used as the sulphur compound and, after the reaction with the halogenated benzotrifluoride, this mixture of sulphur compounds is converted with an acid halide of carbon, phosphorus or sulphur into a compound of the formula

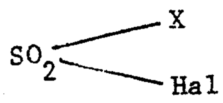

in which

X and Hal have the meaning given in Claim 1, and is removed as such from the reaction mixture.

4. Process according to Claim 1, characterised in that pentachlorobenzotrifluoride is reacted with chlorosulphonic acid at 100 to 130 °C and the pentachlorobenzoyl fluoride formed is then further reacted with potassium fluoride in tetramethylene sulphone at elevated temperature and under increased pressure.

5. Process according to Claims 1 and 3, characterised in that pentachlorobenzotrifluride is reacted with a mixture of sulphuric acid and sulphur trioxide, and, after reaction with thionyl chloride, the sulphuric acid and the oleum, as well as their reaction products, are removed from the reaction mixture by distillation, in the form of compounds of the formula

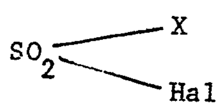

in which

X and Hal have the meaning given in Claim 1.

**Revendications**

1. Procédé de préparation de fluorures de benzoyle halogénés, caractérisé en ce que l'on fait réagir à température élevée un benzotrifluorure halogéné de formule :

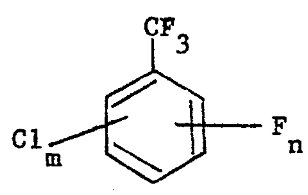

dans laquelle

m et n représentent chacun, indépendamment l'un de l'autre, un nombre entier de 0 à 5, la somme de m et n étant égale à 5, avec au moins un composé du soufre de formule :

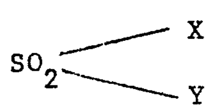

dans laquelle

X représente le fluor, le chlore ou le groupe hydroxy,

Y représente le groupe hydroxy ou bien

X et Y représentent ensemble un atome d'oxygène relié par une double liaison à l'atome de soufre, après la réaction avec le benzotrifluorure halogéné, on élimine du mélange de réaction le composé du soufre sous la forme d'un composé de formule :

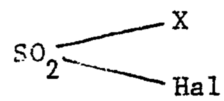

dans laquelle

X a la signification indiquée ci-dessus et

Hal représente le fluor ou le chlore, et le cas échéant, on fait encore réagir le produit de réaction avec le fluorure de potassium à température et pression élevées.

2. Procédé selon la revendication 1, caractérisé en ce que l'on utilise en tant que composé du soufre l'acide chlorosulfonique.

3. Procédé selon la revendication 1, caractérisé en ce que l'on utilise en tant que composé du soufre un mélange d'acide sulfurique et d'anhydride sulfurique, après la réaction avec le benzotrifluorure halogéné, on convertit ce mélange de composés du soufre à l'aide d'un halogénure d'acide du carbone, du phosphore ou du soufre en un composé de formule :

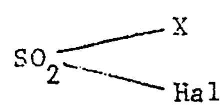

dans laquelle

X et Hal ont les significations indiquées dans la revendication 1, et on l'élimine sous cette forme du mélange de réaction.

4. Procédé selon la revendication 1, caractérisé en ce que l'on fait réagir le pentachlorobenzotrifluorure avec l'acide chlorosulfonique à une température de 100 à 130 °C et on fait ensuite réagir le fluorure de pentachlorobenzoyle obtenu avec le fluorure de potassium dans la tétraméthylènesulfone, à température et pression élevées.

5. Procédé selon les revendications 1 et 3, caractérisé en ce que l'on fait réagir le pentachlorobenzotrifluorure avec un mélange d'acide sulfurique et d'anhydride sulfurique et on élimine du mélange de réaction par distillation l'acide sulfurique et l'oléum ainsi que leurs produits de réaction après réaction avec le chlorure de thionyle, sous forme de composés de formule :

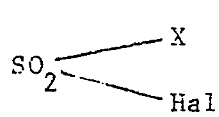

dans laquelle

X et Hal ont les significations indiquées dans la revendication 1.